# EUROPEAN PATENT SPECIFICATION

(11) **EP 3 431 126 B1**
(45) Date of publication and mention of the grant of the patent: **05.08.2026**
(21) Application number: 16894423.9
(22) Date of filing: 17.03.2016
(51) Int. Cl.: A61M 5/32, A61J 1/05, A61M 5/00

(54) **SYRINGE ASSEMBLY, SYRINGE ASSEMBLY PACKAGING, AND PREFILLED SYRINGE**
SPRITZENANORDNUNG, SPRITZENANORDNUNGSVERPACKUNG UND VORGEFÜLLTE SPRITZE
ENSEMBLE SERINGUE, EMBALLAGE D'ENSEMBLE SERINGUE ET SERINGUE PRÉ-REMPLIE

(43) Date of publication of application: 23.01.2019
(73) Proprietor: Terumo Kabushiki Kaisha, Tokyo 151-0072 (JP)
(72) Inventor: TAKEMOTO, Masafumi, Fujinomiya-shi Shizuoka 418-0004 (JP); OKIHARA, Hitoshi, Fujinomiya-shi Shizuoka 418-0004 (JP)
(74) Representative: Casalonga
(86) International application number: PCT/JP2016/058616
(87) International publication number: WO 2017/158805

(56) References cited:
- EP-A1- 2 886 144
- WO-A1-2014/141470
- WO-A1-2014/141470
- WO-A1-2015/091850
- WO-A2-2007/124474
- JP-A- 2008 307 369
- JP-A- S59 200 658
- JP-A- S59 200 658
- JP-A- S62 112 566
- US-A- 4 986 818
- US-A- 4 986 818
- US-A- 5 098 400
- US-A1- 2002 062 108
- US-A1- 2005 038 391
- US-A1- 2016 325 051
- US-B2- 6 719 732

## Description

### Technical Field

The present invention relates to a syringe assembly to which an external cylinder seal cap is mounted, a prefilled syringe using a syringe assembly to which an external cylinder seal cap is mounted, and a packaging storing a plurality of syringe assemblies.

### Background Art

EP2886144A1 relates to a drug delivery device comprising a housing capable of receiving an containing a container and a needle safety device comprising a needle cap arranged to cover and protect a needle, a needle shield relatively movable with respect to the housing to cover and protect the needle and a needle cap remover.

US2002/062108A1 relates to a protection device for a syringe needle comprising an elastic needle cap with a closed distal end and an open proximal end, said cap being formed by a lateral wall defining an inner housing intended to receive the distal part of a needle syringe, and by an end wall capable of being pierced through a part of its thickness by the free end of said needle,

JPS59200658A relates to a medical needle with a cover, which is widely used as a blood transfusion or blood collection side, and aims to provide a medical needle with a cover that is particularly excellent in sterility retention and that facilitates removal of a cover.

WO2014/141470A1 relates to a syringe assembly that comprises an outer cylinder and a seal cap that is mounted to the outer cylinder. The seal cap is provided with: a blocked top section, an open base section, a hollow section, a puncturable section and a protruding section.

A syringe having a puncture needle fixed at a distal end of an external cylinder is used as a syringe for administering a low dose of medical solution, such as an insulin syringe. When a prefilled syringe in which a medical solution is prefilled is configured using this type of syringe, a tip needs to be sealed. Such a seal cap capable of sealing the tip is proposed in, for example, USP 6719732 (Patent Literature 1).

### Citation List

### Patent Literature

Patent Literature 1: USP6719732

### Summary of Invention

### Technical Problem

A seal cap (a device that protects a syringe needle) of Patent Literature 1 (USP 6719732) includes an elastic cap 20 and a rigid shell 80 mounted to the cap 20 as illustrated in Figs. 6 to 10 of Patent Literature 1. It is difficult for a needle 106 to pierce the shell 80. A longitudinal wall 88 of the shell 80 also includes four cut-outs 94 intended to allow passage of water vapor. In addition, the shell 80 includes a cap retaining means for retaining the cap 20 on an inner surface. As the retaining means, a protruding portion 96 protruding inwardly of a cavity is provided.

Typically, when the cap constituted by the rigid shell and the elastic cap as described above is detached from the syringe, the rigid shell and the seal cap are detached together. However, since the seal cap is in close contact with the syringe, when the cap is detached, there is a risk that a hard cover is detached from the seal cap and only the seal cap remains in the state of being mounted to an external cylinder. In particular, when high-pressure steam sterilization (autoclave sterilization) is performed, the seal cap is more likely to be fixed by the syringe due to heat during sterilization, and the above-described risk increases.

Therefore, an object of the present invention is to provide a syringe assembly to which an external cylinder seal cap, a prefilled syringe using a syringe assembly to which an external cylinder seal cap is mounted, and a packaging storing a plurality of syringe assemblies which can reliably remove the entire cap even when being stored in a state where the seal cap is mounted to an external cylinder and even in the case of performing sterilization accompanied with heating such as high-pressure steam sterilization on the syringe assembly to which the seal cap is mounted.

### Solution to Problem

One that achieves the above object is configured as follows.

A syringe assembly according to claim 1. Preferred embodiments are disclosed in the dependent claims.

In addition, one that achieves the above object is configured as follows.

A prefilled syringe including: the above syringe assembly; a gasket stored in the barrel and slidable in the barrel in a liquid-tight manner; and a medical solution filled in a space formed by the barrel and the gasket.

In addition, one that achieves the above object is configured as follows.

A syringe assembly packaging storing a plurality of the above syringe assemblies, the packaging including: a container having an open upper surface and having shape retainability; an external cylinder holding member capable of holding the plurality of syringe assemblies; the plurality of syringe assemblies held by the barrel holding member; and a sheet-shaped lid member that hermetically seals the open upper surface of the container and is peelable. Further, the packaging includes a ventilation portion that is disposed on the container or the lid member and includes bacterial impermeable properties and sterilization gas flowability, and subjected to high-pressure steam sterilization.

### Brief Description of Drawings

Fig. 1 is a front view of a prefilled syringe according to an embodiment of the present invention.
Fig. 2 is a right side view of the prefilled syringe of Fig. 1.
Fig. 3 is a cross-sectional view taken along line A-A of Fig. 1.
Fig. 4 is a partially-omitted enlarged cross-sectional view of a syringe assembly of the present invention used in the prefilled syringe of Figs. 1 and 2.
Fig. 5 is an enlarged front view of an external cylinder seal cap of the present invention used for the prefilled syringe of Figs. 1 to 3 and the syringe assembly of Fig. 4.
Fig. 6 is a cross-sectional view taken along line B-B of Fig. 5.
Fig. 7 is an enlarged right side view of the cylindrical cover member used for the prefilled syringe illustrated in Figs. 1 to 3 and the syringe assembly of Fig. 4.
Fig. 8 is a longitudinal cross-sectional view of the cylindrical cover member of Fig. 7.
Fig. 9 is a cross-sectional view taken along line C-C of Fig. 7.
Fig. 10 is an enlarged plan view of the cylindrical cover member of Fig. 7.
Fig. 11 is an enlarged bottom view of the cylindrical cover member of Fig. 7.
Fig. 12 is a perspective view of the cylindrical cover member illustrated in Fig. 7.
Fig. 13 is an enlarged front view of a seal cap used for the prefilled syringe illustrated in Figs. 1 to 3 and the syringe assembly of Fig. 4.
Fig. 14 is a longitudinal cross-sectional view of the seal cap of Fig. 13.
Fig. 15 is a bottom view of the seal cap illustrated in Fig. 13.
Fig. 16 is a front view of an external cylinder with a puncture needle, which is used for the prefilled syringe illustrated in Figs. 1 to 3 and the syringe assembly of Fig. 4.
Fig. 17 is a perspective view of the barrel with a puncture needle illustrated in Fig. 16.
Fig. 18 is a perspective view of a syringe assembly packaging of the present invention.
Fig. 19 is an explanatory view for describing an internal form of the syringe assembly packaging illustrated in Fig. 18.
Fig. 20 is a front view of the syringe assembly packaging illustrated in Fig. 18.
Fig. 21 is a plan view of the syringe assembly packaging illustrated in Fig. 20.
Fig. 22 is an enlarged cross-sectional view taken along line D-D of Fig. 21.

### Description of Embodiments

A syringe assembly to which an external cylinder seal cap is mounted and a prefilled syringe using a syringe assembly to which an external cylinder seal cap is mounted according to the present invention will be described with reference to embodiments illustrated in the drawings.

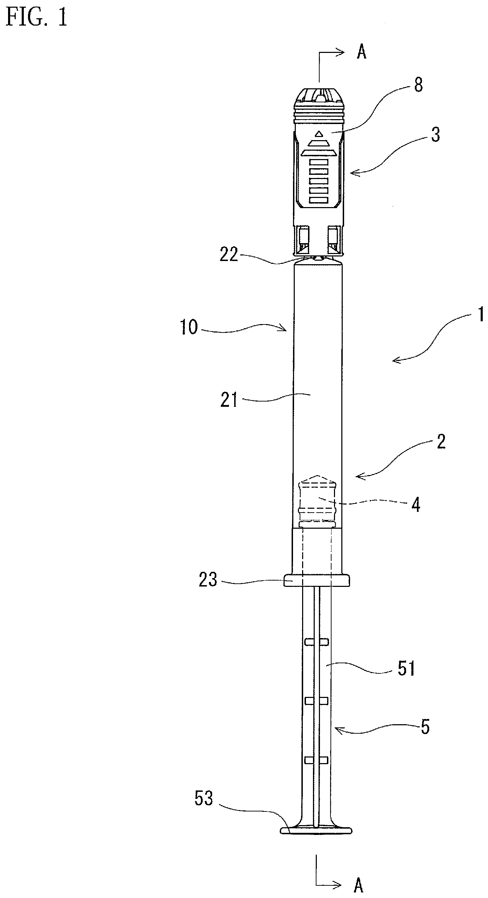

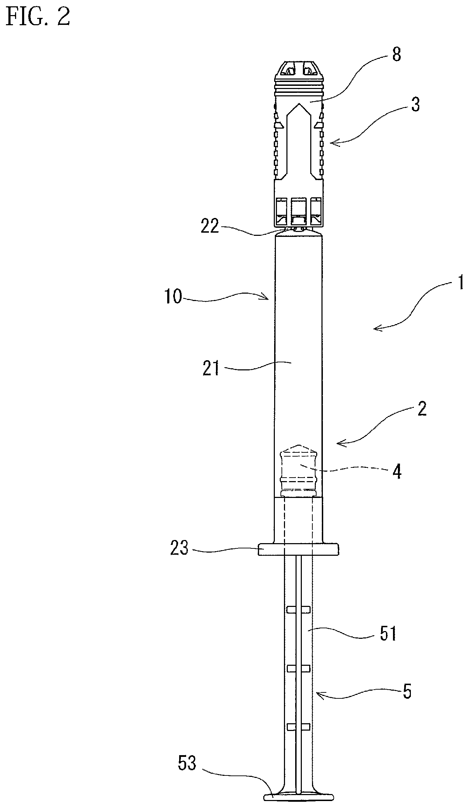

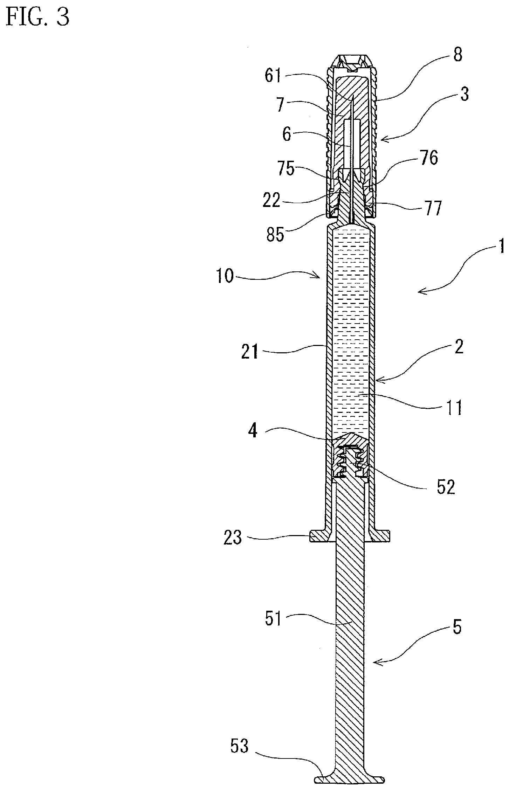

As illustrated in Figs. 1 to 3, a prefilled syringe 1 of the present invention includes a syringe assembly 10, a gasket 4 stored in the syringe assembly 10 and slidable in the syringe assembly 10 in a liquid-tight manner, and a medical solution 11 filled in a space formed of the syringe assembly 10 and the gasket 4.

Further, the syringe assembly 10 of the present invention (in other words, an external cylinder with a puncture needle, to which a cap is mounted) includes an external cylinder 2 and a cap 3 mounted to the barrel 2 as illustrated in Figs. 1 to 4.

The barrel 2 includes an external cylinder body portion 21, a nozzle portion 22 disposed at a distal end of the barrel body portion 21, and a puncture needle 6 including a puncture needle tip 61 at a distal end thereof and a proximal end inserted into and fixed to the nozzle portion 22.

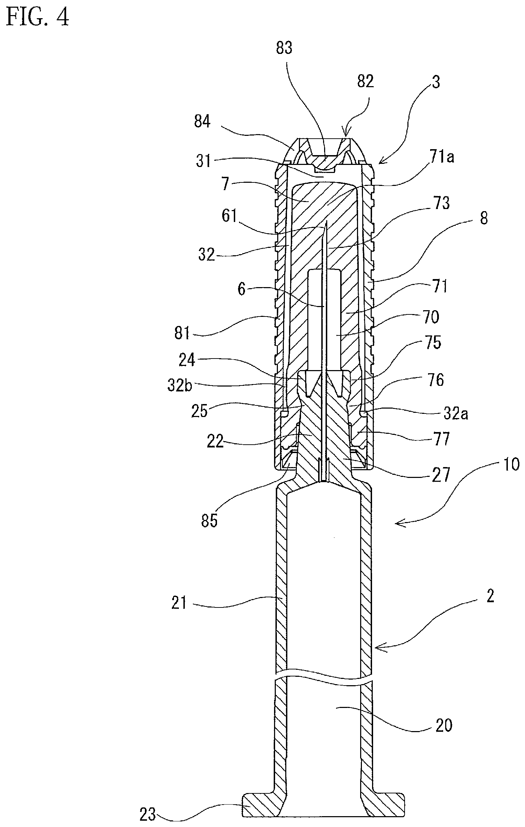

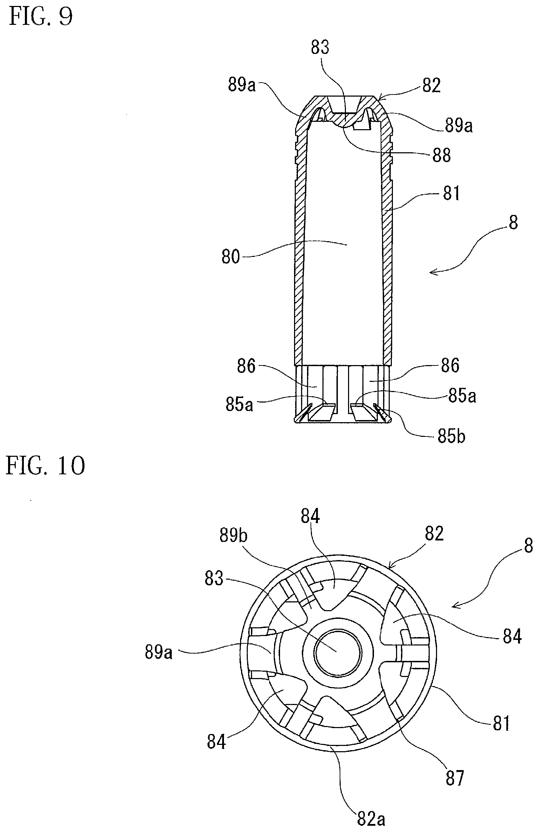

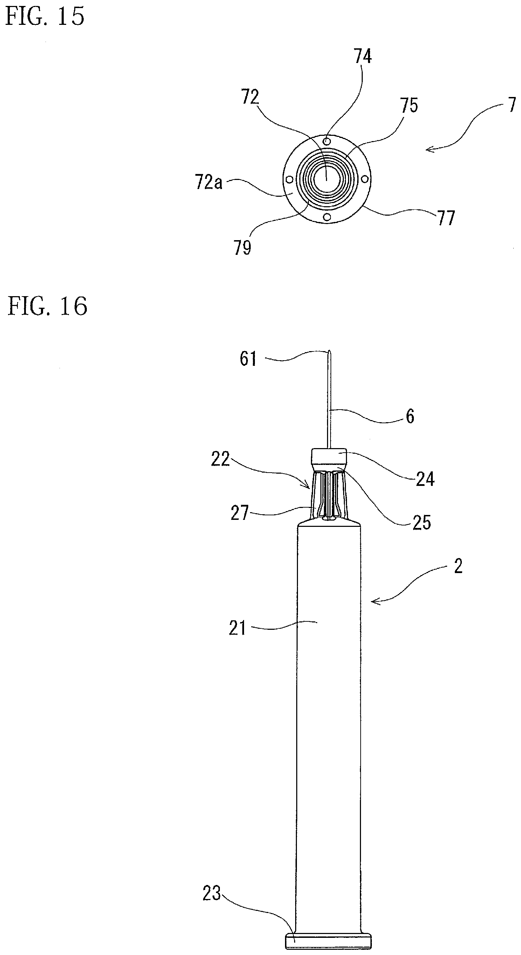

As illustrated in Figs. 4 to 15, the cap 3 includes: a seal cap 7 which includes: a closed distal end portion 71a, an open proximal end portion 72, a hollow portion 70 including a puncture needle storage portion that stores the puncture needle 6, an insertion-allowing portion 73 into which the puncture needle tip 61 of the puncture needle 6 stored in the puncture needle storage portion is insertable, and a seal portion 75 in close contact with the nozzle portion to seal the puncture needle storage portion; and a cylindrical cover member 8 mounted at an outer side of the seal cap 7 to be engaged with the seal cap 7.

Further, the seal cap 7 is made of an elastic material and includes water vapor permeable properties (in other words, of permeable properties of steam for sterilization) for high-pressure steam sterilization (autoclave sterilization). The cylindrical cover member 8 is made of a thermoplastic plastic material harder than the seal cap 7. The cap 3 is configured so that, by high-pressure steam sterilization of the syringe assembly 10, a force of engagement between the cylindrical cover member 8 and the seal cap 7 is increased compared to before sterilization.

As illustrated in Figs. 1 to 3, the prefilled syringe 1 includes: the syringe assembly 10 including the barrel 2 and the cap 3 mounted to the barrel 2 so as to seal the tip of the puncture needle; the gasket 4 stored in the syringe assembly 10 and slidable in the syringe assembly 10 in a liquid-tight manner; the medical solution 11 filled in the space formed by the syringe assembly 10 and the gasket 4; and a plunger 5 attached to the gasket 4 or to be attached at the time of use.

Further, the medical solution 11 is filled in the space formed by the barrel 2, the gasket 4, and the cap 3.

As the medical solution 11 to be filled, any kind of medical solution may be used, and examples thereof may include a high concentration sodium chloride injection solution, minerals, a heparin sodium solution, nitroglycerin, isosorbide dinitrate, cyclosporine, benzodiazepines, antibiotics, vitamin preparations (multivitamin preparations), various amino acids, antithrombotic drugs such as heparin, insulin, antitumor drugs, analgesics, cardiotonics, intravenous anesthetics, an antiparkinsonism drugs, tumor therapeutic drugs, adrenal corticosteroids, arrhythmia drugs, correction electrolytes, antiviral drugs, and immunostimulants.

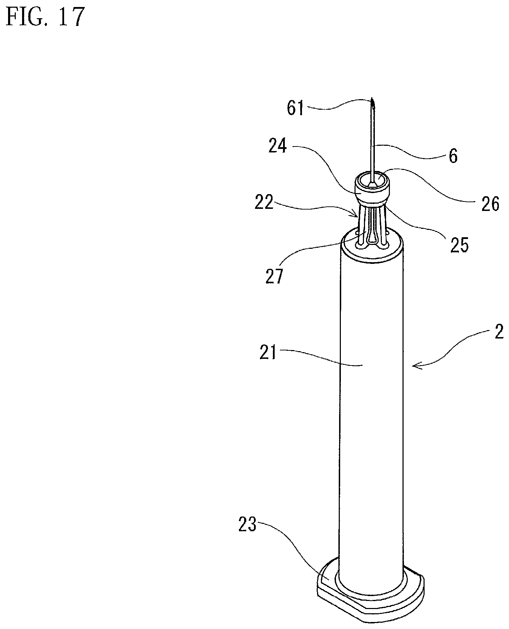

As illustrated in Figs. 1 to 4, 16, and 17, the barrel 2 includes the barrel body portion 21, the cylindrical (hollow) nozzle portion 22 disposed at the distal end of the barrel body portion 21, a flange 23 disposed at a proximal end of the barrel body portion 21, and the puncture needle 6 having the proximal end inserted into and fixed to the nozzle portion 22. The puncture needle 6 includes the puncture needle tip 61 at the distal end thereof. The proximal end of the puncture needle 6 is inserted into and fixed to the hollow portion of the nozzle portion 22 and the inside of the puncture needle 6 communicates with an internal space 20 of the barrel 2. Incidentally, the puncture needle 6 may be inserted into the hollow portion of the nozzle portion 22 of the barrel 2 molded in advance and fixed to the nozzle portion 22 using an adhesive, heat welding, or the like. In addition, the puncture needle 6 may be directly fixed to the barrel 2 by insert-molding. In the case of insert-molding, the nozzle portion 22 forms a cylindrical shape (hollow shape) into which the puncture needle 6 has been inserted by molding the barrel 2, and the puncture needle 6 has the proximal end inserted into and fixed to the hollow portion of the nozzle portion 22.

The barrel 2 is transparent or translucent. The barrel body portion 21 is a substantially cylindrical portion that stores the gasket 4 in a liquid-tight and slidable manner. In addition, the nozzle portion 22 protrudes forward from a distal end (shoulder portion) of the barrel body portion and forms a hollow cylindrical shape with a smaller diameter than the barrel body portion. In addition, as illustrated in Figs. 4, 16, and 17, the nozzle portion 22 includes a head portion 24 disposed at a distal end (in the present embodiment, an annular head portion whose inside is recessed), a short tapered reduced diameter portion 25 disposed at a proximal end of the head portion 24 and having a diameter reduced in a proximal end direction, and a connecting portion 27 connecting a proximal end of the tapered reduced diameter portion 25 and a distal end of the barrel body portion 21, and an annular concave portion is formed by the tapered reduced diameter portion 25.

A recess 26 recessed from a distal end surface toward a proximal end side and a hollow conical portion positioned in the recess 26 and including a vertex on a distal end side are formed in the head portion 24. A plurality of grooves extending in an axial direction of the barrel 2 is formed on an outer surface of the connecting portion 27. Incidentally, the annular concave portion may have a shape which is simply reduced in diameter so that a step is formed between the annular concave portion and the proximal end of the head portion 24, instead of the tapered shape. In addition, the connecting portion 27 may be omitted, and a proximal end of the annular concave portion (the tapered reduced diameter portion 25) and the distal end of the barrel body portion 21 may be directly connected. In addition, the head portion 24 may have a hollow columnar shape (cylindrical shape) in which the recess 26 and the conical portion are omitted.

Examples of a material for forming the barrel 2 may include various resins such as polypropylene, polyethylene, polystyrene, polyamide, polycarbonate, polyvinyl chloride, poly-(4-methylpentene-1), an acryl resin, an acrylonitrile-butadiene-styrene copolymer, polyester such as polyethylene terephthalate, and a cyclic polyolefin polymer, and a cyclic olefin copolymer. Among them, the polypropylene, the cyclic polyolefin (for example, the cyclic olefin polymer and the cyclic olefin copolymer) are preferable since these resins can be easily molded and are heat-resistant.

As the puncture needle 6, a hollow needle including the puncture needle tip 61 at a distal end is used. Metal is generally used as a material for forming the puncture needle 6. As the metal, stainless steel is preferable.

As illustrated in Figs. 1 to 3, the gasket 4 includes a body portion extending with substantially the same outer diameter, and a plurality of annular ribs disposed on the body portion (two ribs in the present embodiment, and an appropriate number of ribs is set as long as the number satisfies the liquid-tightness and the slidability if the number is two or more), and these ribs are brought into contact with the inner surface of the barrel 2 in a liquid-tight manner. In addition, a distal end surface of the gasket 4 has a shape corresponding to a shape of a distal end inner surface of the barrel 2 so as not to form a gap as much as possible between the gasket 4 and the barrel when abutting against the distal end inner surface of the barrel 2.

As a material for forming the gasket 4, it is preferable to use elastic rubber (for example, isoprene rubber, butyl rubber, latex rubber, silicone rubber, or the like), a synthetic resin (for example, a styrene elastomer such as an SBS elastomer and an SEBS elastomer, an olefin elastomer such as an ethylene-α olefin copolymer elastomer, or the like), or the like.

Further, the gasket 4 is disposed with a concave portion extending inward from a proximal end thereof. This recess portion has a female screw shape to be screwable with a male screw portion formed on an outer surface of a protruding portion 52 formed at a distal end of the plunger 5. As the recess portion and the male screw portion are screwed with each other, the plunger 5 is not removed from the gasket 4. Incidentally, the plunger 5 may be disposed in a detached state and attached at the time of use. In addition, the plunger 5 includes the protruding portion 52 protruding in a cylindrical shape forward from a disk portion at the distal end, and a male screw to be screwed with the recess portion of the gasket 4 is formed on the outer surface of the protruding portion. In addition, the plunger 5 includes a body portion 51 extending in an axial direction of a cross-shaped section and a disk portion 53 for pressing disposed at a proximal end.

As illustrated in Figs. 4 to 6, the cap 3 includes the seal cap 7 and the cylindrical cover member 8.

The cap 3 is configured to be used in the state of being mounted to the barrel including: the barrel body portion 21; the nozzle portion 22 disposed at the distal end of the barrel body portion 21 and including the head portion 24 and the annular concave portion 25 formed at the proximal end of the head portion 24; and the puncture needle 6 including the puncture needle tip 61 at the distal end and the proximal end inserted into and fixed to the nozzle portion 22.

The seal cap 7 includes: the closed distal end portion 71a; the open proximal end portion 72; the hollow portion 70 including the puncture needle storage portion that stores the puncture needle 6; the insertion-allowing portion 73 into which the puncture needle tip 61 of the puncture needle 6 stored in the puncture needle storage portion is insertable; and the seal portion 75 in close contact with the nozzle portion to seal the puncture needle storage portion. The cylindrical cover member 8 is mounted on the outer side of the seal cap 7 and engages with the seal cap 7.

The seal cap 7 is made of the elastic material and includes water vapor permeable properties for high-pressure steam sterilization.

As a material for forming the seal cap 7, at least the insertion-allowing portion 73 needs to be made of an elastic material such that the puncture needle is insertable. As an elastic material that allows insertion of the puncture needle and includes water vapor permeable properties for high-pressure steam sterilization, a thermoplastic elastomer, a styrene elastomer such as an SBS elastomer and an SEBS elastomer, an olefin elastomer such as an ethylene-α-olefin copolymer elastomer, or the like is preferable. In addition, rubber such as butyl rubber, isoprene rubber, and silicone rubber may be used as the elastic material.

As illustrated in Figs. 4, and 13 to 16, the seal cap 7 includes a cylindrical body portion 71 and a flange 77 extending from a proximal end of the cylindrical body portion 71 in the proximal end direction. The cylindrical body portion 71 has a form of an outer surface extending in a distal end direction by a predetermined length and having almost the same outer diameter or a diameter reduced slightly in the distal end direction. The flange 77 has an outer diameter larger than the outer diameter of the proximal end of the cylindrical body portion 71 and extends in the proximal end direction by a predetermined length with substantially the same outer diameter.

The cylindrical body portion 71 includes: the closed distal end portion 71a; the seal portion 75 that stores the distal end of the nozzle portion 22; the hollow portion 70 including the puncture needle storage portion that stores the puncture needle; the insertion-allowing portion 73 into which the puncture needle tip 61 of the puncture needle 6 stored in the hollow portion 70 is insertable; and a protruding portion 76 formed on an inner surface of the seal portion 75. Then, when the seal cap 7 is mounted to the nozzle portion 22 of the barrel 2, the puncture needle tip 61 is inserted into the insertion-allowing portion 73 of the seal cap 7 to be sealed, and further, the protruding portion 76 and the annular concave portion 25 of the nozzle portion 22 of the barrel 2 are engaged with each other so that the inner surface of the seal portion 75 and the outer surface of the nozzle portion 22 are brought into close contact with each other to form a sealed state of the puncture needle storage portion. The flange 77 includes an internal space to store a proximal-end-side portion of the nozzle portion 22, the proximal end 72, and a proximal end surface 72a.

Specifically, the seal cap 7 includes the protruding portion 76 disposed on the inner surface positioned on the distal end side of the open proximal end portion 72 by a predetermined length. The protruding portion 76 includes a vertex portion 76a protruding the most and an inclined portion (tapered portion) 76b extending in the distal end direction from the vertex portion 76a and having a protrusion height gradually decreasing in the distal end direction. In particular, in the present embodiment, the protruding portion 76 is an annular protruding portion, and the inclined portion 76b is a tapered portion in which an inner diameter of the seal portion 75 decreases in the distal end direction.

The inner diameter of the seal portion 75 at the vertex portion 76a is slightly smaller than the outer diameter of the distal end of the annular concave portion 25 of the nozzle portion 22 of the barrel 2. As a result, when the seal cap 7 is mounted to the nozzle portion 22 of the barrel 2, the protruding portion 76 and the annular concave portion 25 are engaged with each other. In addition, the distal-end-side inclined portion 76b extends to the distal end side of the annular concave portion 25 in a state where the seal cap 7 is mounted to the nozzle portion 22 of the barrel 2. Further, the inner diameter of the seal portion 75 in the vicinity of at least the proximal end of the distal-end-side inclined portion 76b is slightly smaller than the outer diameter of the head portion 24 of the nozzle portion 22 of the barrel 2. As a result, when the seal cap 7 is mounted to the nozzle portion 22 of the barrel 2, the distal-end-side inclined portion 76b is pressed against the outer surface of the head portion 24 to be brought into close contact with the inclined portion 76b, and thus, undesirable removal of the seal cap 7 from the barrel 2 is further reduced.

In particular, in the present embodiment, the seal portion 75 of the seal cap 7 is in the state of being expanded outward by the nozzle portion 22 of the stored external cylinder 2 as illustrated in Fig. 4. Specifically, the seal portion 75 is in the state of being elastically deformed and slightly inflated. Thus, the seal portion 75 is formed such that a distance from the inner surface of the cylindrical cover member 8, which will be described later, is shorter than those of the other portions, thereby regulating inflow of an ozone gas under the atmospheric pressure at the time of manufacturing or the like.

In addition, the protruding portion 76 is formed to be annular along the inner surface of the seal portion 75 in the seal cap 7 of the present embodiment. Thus, the area of the distal-end-side inclined portion 76b, which is pressed against the outer surface of the head portion 24 be brought into close contact therewith increases as compared to the case where the protruding portions 76 are formed at intervals on the inner surface of the seal portion 75, and the undesirable removal of the seal cap 7 from the barrel 2 is further reduced. Incidentally, the protruding portions 76 may be formed at intervals on the inner surface of the seal portion 75.

In addition, in the seal cap 7 of the present embodiment, the protruding portion 76 further includes a proximal-end-side inclined portion 76c which extends in an open end (proximal end) direction from the vertex portion 76a and whose protruding height gradually decreases in the open end (proximal end) direction. As a result, when the seal cap 7 is mounted to the nozzle portion 22 of the barrel 2, the vertex portion 76a of the protruding portion 76 easily gets over the head portion 24 of the nozzle portion 22 from the distal end side.

In particular, in the present embodiment, the protruding portion 76 is the annular protruding portion, and the proximal-end-side inclined portion 76c is a proximal-end-side tapered portion in which the inner diameter of the seal portion 75 increases in the proximal end direction. Incidentally, in the seal cap 7 of the present embodiment, the proximal-end-side inclined portion (proximal-end-side tapered portion) 76c is shorter than the distal-end-side inclined portion (distal-end-side tapered portion) 76b and has a larger taper angle.

In addition, in the seal cap 7 of the present embodiment, the seal portion 75 includes a linear portion 76d extending in the distal end direction from the distal end of the distal-end-side inclined portion 76b of the protruding portion 76 by a predetermined length (specifically, to the proximal end of the hollow portion 70). The inner diameter of the seal portion 75 at the linear portion 76d is constant and slightly smaller than the outer diameter of the head portion 24 of the nozzle portion 22 of the barrel 2. Thus, when the seal cap 7 is mounted to the nozzle portion 22 of the barrel 2, the linear portion 76d is pressed against the outer surface of the head portion 24 to be brought into close contact therewith Incidentally, the inner diameter of the seal portion 75 in the linear portion 76d may be larger than the outer diameter of the head portion 24 of the nozzle portion 22 of the barrel 2. In addition, the linear portion 76d may be omitted and the distal-end-side inclined portion 76b may be extended to the proximal end of the seal portion 75.

Further, a removal resistance of the seal cap 7 from the barrel 2 is preferably 1.5N to 20 N, and particularly preferably 5 to 8 N. As a result, it is possible to easily remove the seal cap 7 from the barrel 2 at the time of use of the prefilled syringe 1 while preventing the undesirable removal of the seal cap 7 from the barrel 2.

In addition, an inclination angle (taper angle) of the distal-end-side inclined portion 76b of the protruding portion 76 of the seal cap 7 is preferably 1 to 10 degrees, and particularly preferably 1 to 6 degrees. In addition, the protruding height of the vertex portion of the protruding portion 76 is preferably 0.1 to 0.5 mm, and particularly preferably 0.05 to 0.25 mm.

In addition, in the present embodiment, the proximal end of the distal-end-side inclined portion 76b of the protruding portion 76 of the seal cap 7 is positioned around the annular concave portion 25 of the nozzle portion 22 of the barrel 2, and the inner diameter of the seal portion 75 at least in the vicinity of the proximal end of the distal-end-side inclined portion 76b is slightly smaller than the outer diameter of the annular concave portion 25. In addition, the annular concave portion 25 of the barrel 2 is formed of the tapered reduced diameter portion disposed at the proximal end of the head portion 24 and whose diameter decreases in the proximal end direction in the present embodiment. As a result, when the seal cap 7 is removed from the barrel 2, the protruding portion 76 of the seal cap 7 is expanded outward along the annular concave portion 25 to easily get over the head portion 24.

Further, a length, in the axial direction of the head portion 24, of a portion of the distal-end-side inclined portion 76b of the protruding portion 76 of the seal cap 7, the portion pressed against the outer surface of the head portion 24 to be brought into close contact therewith is preferably 0.1 to 2.0 mm, and particularly preferably 0.3 to 1.5 mm. As a result, it is possible to reduce the undesirable removal of the seal cap 7 from the barrel 2, and to suppress the removal resistance of the seal cap 7 from the barrel 2 from being unnecessarily large.

Further, the seal cap 7 of the present embodiment includes a nozzle portion introduction portion 78 which is formed from the open proximal end portion 72 of the seal cap 7 to the proximal end of the seal portion 75 (the protruding portion 76) and extends with substantially the same inner diameter. The nozzle portion introduction portion 78 has the inner diameter slightly larger than the maximum inner diameter of the seal portion 75 and is slightly larger than the outer diameter of the head portion 24 of the nozzle portion 22 of the barrel 2. Thus, the seal cap 7 functions as an introduction portion of the nozzle portion 22 when the seal cap 7 is mounted to the nozzle portion 22 of the barrel 2. In addition, the nozzle portion introduction portion 78 includes an annular rising surface 79 erected toward the open proximal end portion 72 at a boundary with the proximal end of the seal portion 75 (the protruding portion 76).

Accordingly, when the distal end of the barrel 2 is inserted into the nozzle portion introduction portion 78 of the seal cap 7, the nozzle portion 22 of the barrel 2 has entered the inside of the nozzle portion introduction portion 78, and then, the annular distal end surface of the head portion 24 of the nozzle portion 22 abuts against the annular rising surface 79. In this state, the puncture needle 6 becomes substantially parallel to a central axis of the seal cap 7 and is in the state of entering the inside of the hollow portion 70.

In addition, the seal cap 7 of the present embodiment is configured such that the puncture needle tip 61 of the puncture needle 6 enters the inside of the hollow portion 70 not to reach the insertion-allowing portion 73 in the state where the nozzle portion 22 of the barrel 2 is inserted into the seal cap and the annular distal end surface of the head portion 24 of the nozzle portion 22 abuts against the annular rising surface 79 of the nozzle portion introduction portion 78 of the seal cap 7.

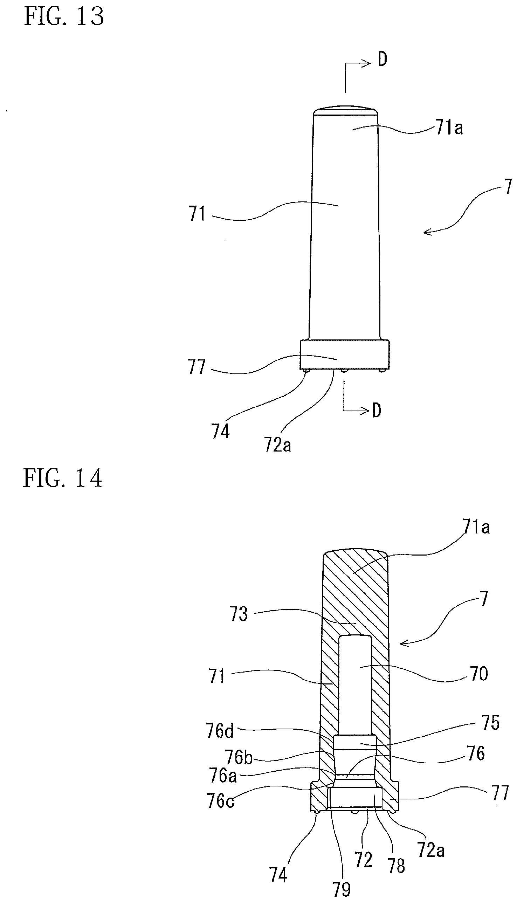

In addition, the flange 77 protruding annularly outward is formed at the proximal end of the seal cap 7. A distal-end-side position of the flange 77 is positioned on the distal end side of the annular rising surface 79 of the hollow portion 70 and positioned in the vicinity of the vertex portion 76a of the protruding portion 76 (slightly closer to the proximal end opening portion 72 side than the vertex portion 76a in the case illustrated in Figs. 4 and 14) .

Further, the open proximal end portion 72 of the seal cap 7 includes the annular proximal end surface 72a, and a plurality of protrusions 74 is disposed on the proximal end surface 72a. Further, the proximal end surface 72a forms an engagement portion on the seal cap side at the time of engagement with the cylindrical cover member 8. The proximal end surface 72a also serves as a proximal end surface of the flange 77. Accordingly, it can be also said that the cap-side engagement portion is formed by the proximal end surface 72a and the flange 77. Incidentally, the proximal end surface of the flange 77 may be disposed on the distal end side of the open proximal end portion 72. In this case, the cap-side engagement portion is formed by not the proximal end surface 72a but the proximal end surface of the flange 77.

Next, the cylindrical cover member 8 will be described with reference to Figs. 4 to 12.

The cylindrical cover member 8 is made of a thermoplastic plastic material harder than the seal cap 7, and is configured so that, by high-pressure steam sterilization of the syringe assembly 10, the force of engagement between the cylindrical cover member 8 and the seal cap 7 is increased compared to before sterilization.

As illustrated in Figs. 4 to 12, the cylindrical cover member 8 is a cylindrical body including a seal cap storage portion 80 at the inside thereof. The cylindrical cover member 8 of the present embodiment includes a side surface covering portion 81 that covers a side surface of the seal cap 7 and a distal end covering portion 82 that covers the closed distal end portion of the seal cap 7.

Further, the cylindrical cover member 8 includes an engagement mechanism for regulation of removal which is configured in cooperation with the seal cap 7. According to the invention, the cylindrical cover member 8 includes an internal protruding portion disposed on the inner surface of the cylindrical cover member 8 to be engaged with the seal cap 7. Further, this internal protruding portion is displaced inward by heat of high-pressure steam sterilization, whereby the force of engagement between the cylindrical cover member 8 and the seal cap 7 is increased compared to before sterilization. In addition, the internal protruding portion extends obliquely from the inner surface of the cylindrical cover member 8 in a center axis direction of the cylindrical cover member 8 and in the distal end direction, and is elastically deformable. When the cylindrical cover member 8 is mounted to the seal cap 7, the internal protruding portion is pushed by the outer surface of the seal cap and is elastically deformed in a direction away from the center axis of the cylindrical cover member 8, and thus, it is easy to mount the cylindrical cover member 8 to the seal cap 7. in addition, when the cap 3 is removed from the barrel 2, a distal end of the internal protruding portion is pushed by the seal cap 7 to be deformed in a direction approaching the central axis of the cylindrical cover member 8, and thus, it is difficult for the cylindrical cover member 8 to be removed from the seal cap 7. In addition, a plurality of the internal protruding portions is disposed at intervals about the central axis of the cylindrical cover member 8. Specifically, the cylindrical cover member 8 of the present embodiment includes a plurality of internal protruding portions 85 (85a and 85b) disposed on the inner surface of the proximal end of the side surface covering portion 81. In particular, in the illustrated embodiment, the plurality of elastically-deformable internal protruding portions 85a and 85b extending obliquely from the inner side of the proximal end (substantially the proximal end) of the side surface covering portion 81 in the central axis direction and in the distal end direction of the cylindrical cover member 8 and including free ends at positions which do not reach the center of the cylindrical cover member 8. Further, the free ends of the respective internal protruding portions 85 (85a and 85b) are capable of abutting against the proximal end surface 72a or the protrusion 74 of the seal cap 7.

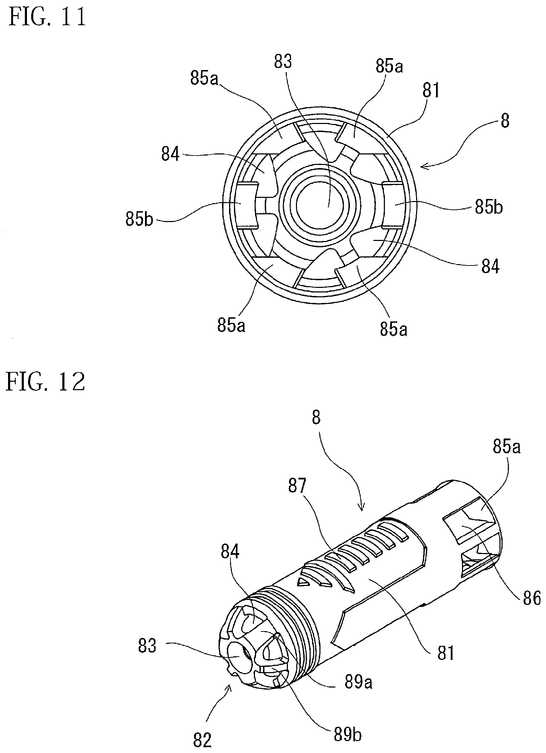

Further, a width of each of the four internal protruding portions 85a is narrowed toward the free end as illustrated in Fig. 11. In particular, the internal protruding portion 85a has one side surface being inclined so that the width is narrowed toward the free end. In addition, the two internal protruding portions 85a and 85b disposed so as to face each other extend to the free ends with the same width. In addition, an inner surface of the free end of each of the internal protruding portions 85a and 85b of the cylindrical cover member 8 is preferably an arcuate surface having the central axis of the cylindrical cover member 8 substantially as the center. Further, in the present embodiment, the free ends of the internal protruding portions 85a and 85b have a certain degree of circular arc length and can reliably abut against the proximal end surface 72a or the protrusion 74 of the seal cap 7.

In addition, when the cylindrical cover member 8 is mounted on the seal cap 7, the free ends of the internal protruding portions 85a and 85b are deformed in the direction away from the center axis of the cylindrical cover member. In addition, when the cylindrical cover member 8 moves in the direction to be removed from the seal cap 7, the free ends of the internal protruding portions 85a and 85b are deformed in the direction approaching the central axis of the cylindrical cover member. Further, in the cylindrical cover member 8 of the present embodiment, rear-end-side surfaces of the plurality of internal protruding portions 85a and 85b of the cylindrical cover member 8 are inclined surfaces that are smooth and faces toward the center of the cylindrical cover member, and form a guide portion configured to guide the seal cap 7. In addition, in the cylindrical cover member 8 of the present embodiment, the distal-end-side surfaces of the plurality of internal protruding portions 85a and 85b of the cylindrical cover member 8 are also inclined surfaces that are smooth and face toward the center of the cylindrical cover member. Further, each of the internal protruding portions 85a and 85b becomes thinner toward the free end, and has elastic deformability that is increased toward the free end.

Further, an outer diameter of the maximum outer diameter portion (flange) of the seal cap 7 is larger than a diameter of a circle formed by the inner surface of the free end of each of the internal protruding portions 85a and 85b of the cylindrical cover member 8 preferably by 0.5 to 5 mm, and particularly preferably by 1.5 to 3 mm. In addition, it is preferably that the inner diameter of the proximal end of the side surface covering portion 81, in other words, the inner diameter of the portion storing the flange 77 of the seal cap 7 be substantially equal to the outer diameter of the flange 77 of the seal cap 7. In particular, the proximal end of the side surface covering portion 81 is preferably in close contact with the flange 77 of the seal cap 7 after the high-pressure steam sterilization. In particular, the proximal end of the side surface covering portion 81 is preferably in close contact with and pressed against the flange 77 of the seal cap 7 after the high-pressure steam sterilization.

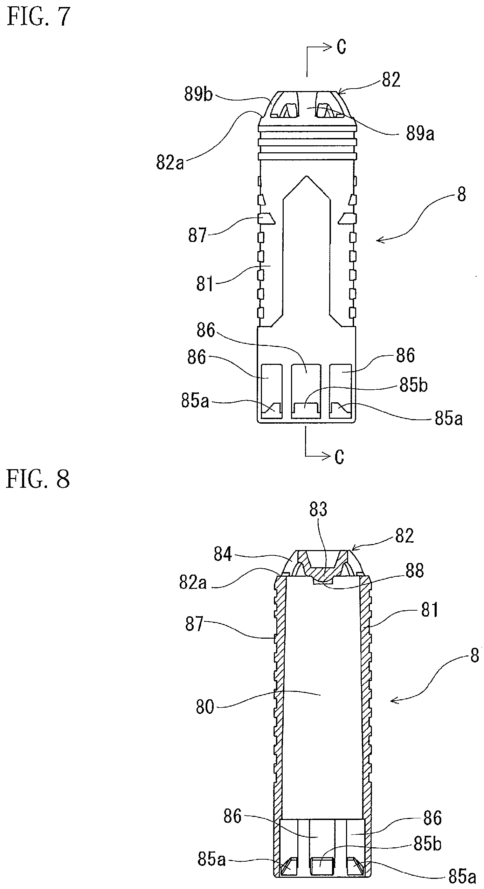

Further, in the cylindrical cover member 8 of the present embodiment, an opening 86 extending in the distal end direction from the proximal end of each of the internal protruding portions 85a and 85b is provided as illustrated in Figs. 7 to 9. As a result, the internal protruding portions 85a and 85b are more easily deformed in the direction away from the center axis of the cylindrical cover member 8, and it is easy to mount the cylindrical cover member 8 to the seal cap 7. Further, the six internal protruding portions (specifically, the four internal protruding portions 85a and the two internal protruding portions 85b) are arranged at substantially equal angles with respect to the central axis of the cylindrical cover member 8 in the present embodiment.

Further, the cylindrical cover member 8 of the present embodiment includes a through-hole 84 disposed at the distal end, specifically, in the distal end covering portion 82 as illustrated in Figs. 4 to 12. Further, the cap 3 includes a steam guide gap 32 which is formed by the inner surface of the cylindrical cover member 8 and the outer surface of the seal cap 7, communicates with the through-hole 84, and extends to the proximal end of the puncture needle storage portion of the seal cap 7 (in other words, the distal end of the flange). In the present embodiment, the steam guide gap 32 extends from the through-hole 84 to the distal end of the flange 77 of the seal cap 7. In addition, the steam guide gap 32 is an annular space so as to surround the outer surface of the seal cap 7.

In addition, the steam guide gap 32 communicates with the opening 86 at a proximal end 32a thereof. Specifically, a part of the proximal end (in other words, a portion closer to the distal end side than the flange 77) of the cylindrical body portion 71 of the seal cap 7 is positioned at the distal end of the opening 86, and in this portion, a gap 32a is formed between the outer surface of the seal cap 7 (specifically, the outer surface of the proximal end of the cylindrical body portion 71 of the seal cap 7) and the inner surface of the proximal end of the cylindrical cover member 8. As a result, the steam guide gap 32 has the distal end communicating with the through-hole 84, and the proximal end communicating with the opening 86. Thus, the flow of steam for sterilization becomes favorable. Incidentally, the proximal end 32a of the steam guide gap 32 may not necessarily communicate with the opening 86. In this case, the flow of the steam for sterilization is secured due to a pressure fluctuation at the time of high-pressure steam sterilization.

Further, in the present embodiment, the seal portion 75 of the seal cap 7 is in the state of being expanded outward by the nozzle portion 22 of the stored external cylinder 2 as illustrated in Fig. 4. Specifically, the seal portion 75 is in the state of being elastically deformed and slightly inflated. Thus, the steam guide gap in this portion is a narrow portion 32b having a narrower width than the other portion of the steam guide gap 32, and regulates inflow of an ozone gas under atmospheric pressure at the time of manufacturing or the like.

The width of the steam guide gap 32 (a distance between the inner surface of the cylindrical cover member 8 and the outer surface of the seal cap 7) is preferably 0.1 to 2 mm. The flowability of the steam for sterilization deteriorates if the width of the steam guide gap 32 is smaller than 0.1 mm, and wobbling of the cylindrical cover member 8 with respect to the seal cap 7 is large so that the operability at the time of detaching the cap 3 from the barrel 2 deteriorates if the width is larger than 2 mm. In addition, the narrow portion 32b is narrower than the width of the other portion of the steam guide gap 32 (the distance between the inner surface of the cylindrical cover member 8 and the outer surface of the seal cap 7) preferably by 0.1 to 2 mm.

Further, in the cylindrical cover member 8 of the present embodiment, the distal end covering portion 82 includes a plurality of frames connected to the distal end of the side surface covering portion 81, and the through-hole 84 is formed between adjacent frames. More specifically, the distal end covering portion 82 includes: a distal end central portion 83 including a concave portion at the center; the plurality of frames connecting the distal end central portion 83 and the distal end of the side surface covering portion 81; and the plurality of through-holes 84 each of which is formed between the frames. In particular, the distal end covering portion 82 includes six frames 89a and 89b in the present embodiment. Further, the frame 89a is thick, and the frame 89b is thin. In addition, the frames 89a and 89b are alternately arranged three by three. In addition, all the frames 89a and 89b are disposed so as to be substantially equiangular with respect to the center axis of the cylindrical cover member 8. In addition, the number of the through-holes is six in the present embodiment, but is preferably about 2 to 10 and particularly preferably 3 to 8. In addition, the total area of the through-holes in a plan view of the distal end side of the cylindrical cover member 8 is preferably 3 to 25 mm², and particularly preferably 5 to 20 mm². With such a configuration, the distal end maintains sufficient strength and allows favorable passage of the steam for sterilization therethrough.

Further, in the cylindrical cover member 8, a corner portion 82a formed at a boundary portion between the proximal ends of the frames 89a and 89b and the distal end of the side surface covering portion 81 and faces the distal end direction in a side view illustrated in Fig. 7. A width of the corner portion 82a is preferably 0.2 to 1 mm. As such a corner portion 82a is disposed, the syringe assembly 10 is easily inserted into a cylindrical portion 142 of the barrel holding member 104 to be described later or a hole of a centering plate to be used at the time of filling a drug solution while securing a size of the through-hole. In addition, the cylindrical cover member 8 includes multiple anti-slip ribs 87 formed on an outer surface as illustrated in the drawings.

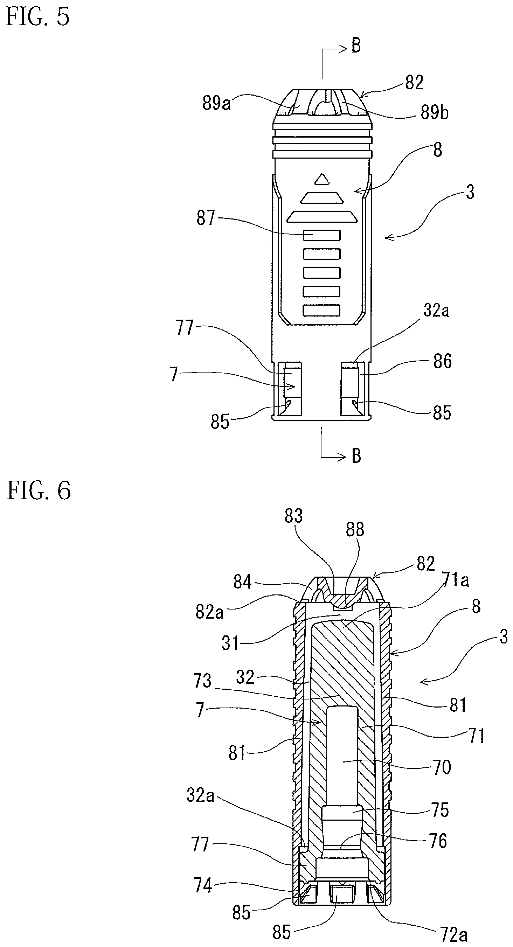

Further, the cylindrical cover member 8 includes an opposing inner surface 88 that opposes the distal end surface of the closed distal end portion 71a of the seal cap 7 and a gap 31 is formed between the opposing inner surface 88 and the closed distal end portion 71a in the cap 3 of the present embodiment as illustrated in Figs. 4 and 6. Thus, the flow of water vapor inside the cap becomes favorable. In addition, condensed water at the time of sterilization is hardly trapped so that a drying property becomes favorable. In particular, at the time of performing sterilization with the distal end of the cap 3 facing downward, condensed water tends to collect at the tip portion, which is effective. A length of the gap 31, in other words, a distance between the opposing inner surface 88 and the closed distal end portion 71a is preferably 0.1 to 5 mm, and particularly preferably 0.3 to 2 mm.

Incidentally, in the above-described structural characteristics of the cylindrical cover member 8, the syringe assembly (in other words, the barrel with a puncture needle, to which the cap is mounted) 10 has an excellent effect regardless of whether the force of engagement between the cylindrical cover member 8 and the seal cap 7 after the high-pressure steam sterilization is increased compared to before sterilization or not. That is, for example, in the cap 3 illustrated in Figs. 5 and 6, the cylindrical cover member 8 is engaged with the proximal end (flange 77) of the seal cap 7 at the proximal end, and thus, has a sufficient force of engagement even before sterilization. Further, the cap 3 includes the gap 32 formed between the cylindrical cover member 8 and the seal cap 7 and the through-hole 84 communicating with the gap 32. Thus, in the case of EOG sterilization, since an ethylene oxide gas flows from the through-hole 84 to the gap 32, the gas flowability is favorable. In addition, the width of the corner portion formed at the distal end of the cylindrical cover member is 0.2 to 1 mm as described above, and thus, the syringe assembly can be easily inserted into the cylindrical portion of the cylindrical cover member or the hole of the centering plate to be used at the time of filling the drug solution while securing the size of the through-hole 84.

In addition, in the case where the cap is of the above-described type, it is preferable that the cylindrical cover member 8 and the seal cap 7 have a sufficient force of engagement before sterilization. In addition, in this case, a sterilization method other than the high-pressure steam sterilization such as EOG sterilization, radiation sterilization may be used as the sterilization method. Incidentally, the seal cap 7 may be one which does not include water vapor permeable properties (permeable properties of steam for sterilization) for high-pressure steam sterilization. In addition, in the case of using the EOG sterilization, one including permeable properties of the ethylene oxide gas is used as the seal cap 7.

As a material for forming the cylindrical cover member 8, a material harder than the material for forming the seal cap 7 is used. Examples of the forming material may include various resins such as polyesters such as polypropylene, polyethylene, polystyrene, polyamide, polycarbonate, polyvinyl chloride, poly (4-methylpentene-1), an acrylic resin, an acrylonitrile-butadiene-styrene copolymer, polyester such as polyethylene terephthalate, and cyclic polyolefins.

Further, the cylindrical cover member 8 preferably has heat shrinkability depending on heat during the high-pressure steam sterilization. The cap 3 thermally shrinks due to the heat during high-pressure steam sterilization. As a result, the force of engagement between the cylindrical cover member 8 and the seal cap 7 is increased more than before sterilization. Incidentally, as long as the form of the cylindrical cover member 8 at the time of molding includes the gap 32 between the cylindrical cover member 8 and the outer surface of a portion of the seal cap 7 excluding the flange 77 as illustrated in Figs. 4 and 6, the gap 32 which communicates with the through-hole 84 and through which water vapor during the high-pressure steam sterilization can pass is maintained between the inner surface of the cylindrical cover member 8 and the outer surface of the seal cap 7 as illustrated in Fig. 4.

Further, the cylindrical cover member 8 is preferably mounted to the seal cap 7 after the seal cap 7 is mounted to the barrel 2. When only the seal cap is mounted at an early stage in this manner, it can be confirmed whether the puncture needle is inserted straight into the seal cap. The seal cap bends if the puncture needle is inserted obliquely, and thus, the state of the puncture needle can be easily confirmed. However, the cap does not bend in the cap to which the cylindrical cover member has been mounted even when the needle is bent and inserted so that it is difficult to confirm the state of the puncture needle. Further, the syringe assembly 10 is preferably subjected to the high-pressure steam sterilization.

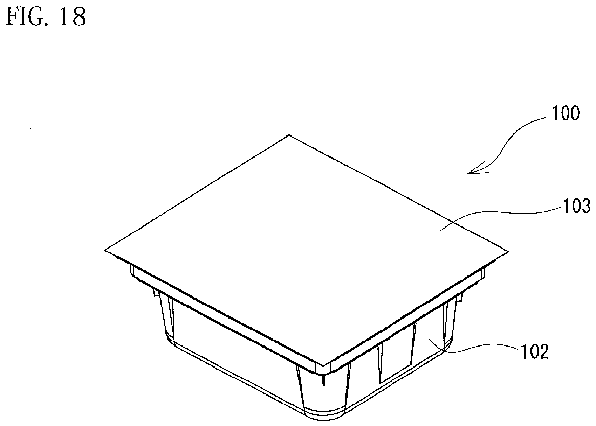

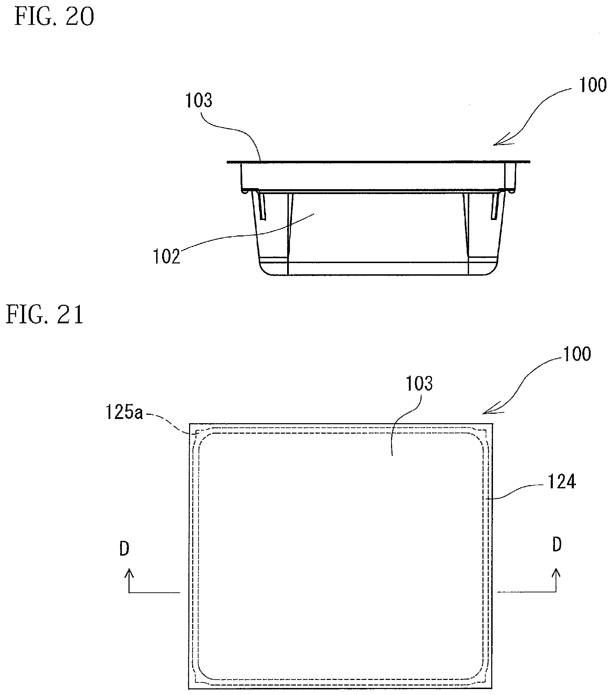

Next, an embodiment illustrated in the drawings will be described with reference to Figs. 18 to 22 regarding a packaging storing a plurality of the syringe assemblies of the present invention.

A sterilizable or sterilized prefilled syringe assembly packaging 100 storing the plurality of syringe assemblies of the present invention includes: a container 102 having an open upper surface and having shape retainability; an external cylinder holding member 104 capable of holding the plurality of syringe assemblies 10 stored in the container 102; the plurality of syringe assemblies 10 held by the barrel holding member 104; and a sheet-shaped lid member 103 which hermetically seals the open upper surface of the container 102 and is peelable.

The prefilled syringe assembly packaging 100 of the present invention is a sterilizable or sterilized prefilled syringe assembly packaging. High-pressure steam sterilization is used as a sterilization method.

As illustrated in Figs. 18, 19, and 22, the prefilled syringe assembly packaging 100 of the present invention includes: the container 102; the barrel holding member 104 capable of holding the plurality of syringe assemblies 10; the plurality of syringe assemblies 10 held by the barrel holding member 104; and the sheet-shaped lid member 103 which hermetically seals the open upper surface of the container 102 and is peelable. Further, the packaging 100 includes a ventilation portion disposed on the container 102 or the sheet-shaped lid member 103 and including bacterial impermeable properties and sterilization gas flowability.

As illustrated in Figs. 18 to 22, the container 102 is of a tray-shaped container having a predetermined depth and a certain degree of strength and shape retainability, and includes: a body portion 121, an external cylinder holding member holding portion 126 formed at an upper portion of the body portion 121 and configured to hold a peripheral edge of the barrel holding member 104 holding the plurality of syringe assemblies 10, and an annular flange 124 disposed on the open upper surface.

Further, an annular heat-sealing convex portion 125 for fixing with the sheet-shaped lid member 103 is disposed on an upper surface of the annular flange 124. Further, the barrel holding member holding portion 126 is formed at a position on a bottom surface side by a predetermined length from the flange 124. In the container 102 of this first embodiment, the barrel holding member holding portion 126 is an annular step portion such that the peripheral edge of the barrel holding member 104 holding the plurality of syringe assemblies 10 can be placed.

The container 102 preferably has a certain degree of shape retainability and rigidity. In addition, it is desirable to use a thermoplastic material having heat resistance (120°C or higher) in order to cope with the high-pressure steam sterilization. As Examples of a material having a certain degree of shape retainability, a certain degree of rigidity, heat resistance, and thermoplasticity include polyolefin such as polypropylene and polyethylene, a vinyl chloride resin, a polystyrene/polypropylene resin, polyethylene/ionomer (for example, ethylene-based, styrene-based, fluorine-based)/polyethylene, a polyester resin (for example, polyethylene terephthalate, polybutylene terephthalate, amorphous polyethylene terephthalate), and PP/EVOH/PP (laminate). In this case, a thickness of the container 102 is preferably about 0.05 to 4.00 mm, and particularly, more preferably 1.00 to 2.00 mm.

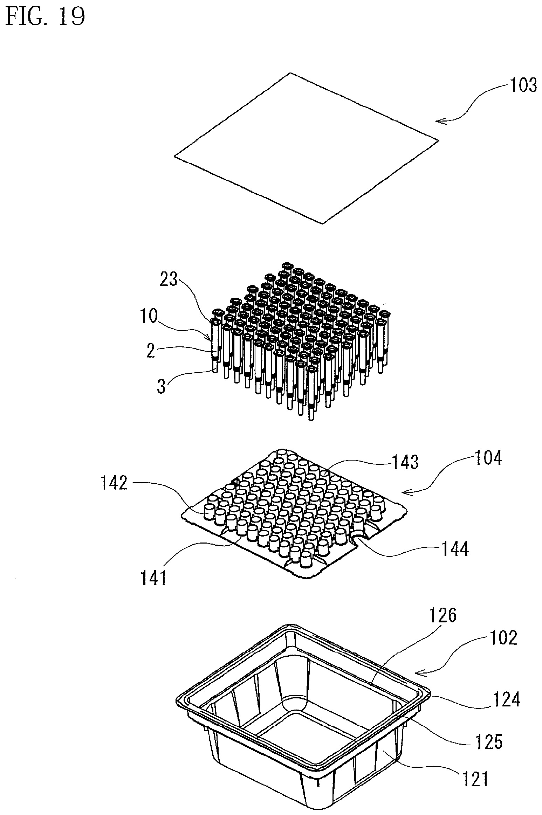

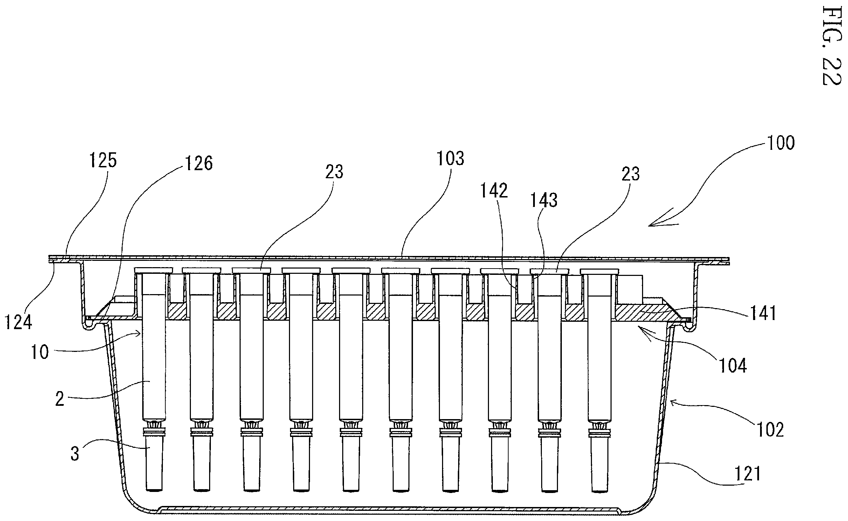

As illustrated in Figs. 19 and 22, the barrel holding member 104 capable of holding the plurality of syringe assemblies 10 includes a substrate portion 141 and a plurality of cylindrical portions 142 protruding upward from the substrate portion 141. Further, an external cylinder holding opening 143 is formed in the cylindrical portion 142 and a cutout portion 144 for grip is formed on a side portion of the substrate portion 141. An inner diameter of the cylindrical portion 142 and the barrel holding opening 143 is larger than an outer diameter of the largest diameter portion of the syringe assembly 10 to be held, and is set to prevent passage of the flange portion 23 of the syringe assembly 10 to be held.

Thus, the syringe assembly 10 penetrates through the cylindrical portion 142 and the flange 23 of the syringe assembly 10 is in the state of being suspended by the barrel holding opening 143 as illustrated in Fig. 22. In addition, a lower end (the distal end of the seal cap 7) of the syringe assembly 10 held by the barrel holding member 104 is not in contact with a bottom surface of the container 102 as illustrated in Fig. 22. In other words, the bottom surface of the container 102 and the lower end of the syringe assembly 10 held by the barrel holding member 104 (the distal end of the seal cap 7) are separated from each other and do not inhibit the flow of water vapor. It is desirable that a material for forming the barrel holding member 104 also include heat resistance (120°C or higher) in order to cope with the high-pressure steam sterilization.

As the sheet-shaped lid member 103, it is desirable to use a member to which microparticles such as bacteria and viruses are impermeable for high-pressure steam sterilization and water vapor is permeable. In addition, a member capable of heat-sealing of the container 102 is preferable. As the sheet-shaped lid member 103, for example, a synthetic resin nonwoven fabric, specifically, a nonwoven fabric made of a synthetic resin material such as polyolefin known as Tyvek (registered trademark), a synthetic resin porous membrane, and the like can be suitably used.

Further, the sheet-shaped lid member 103 is heat-sealed against the heat-sealing convex portion 125 disposed on the annular flange 124 of the container 102 such that a peripheral edge thereof is peelable. Incidentally, an outer edge of the sheet-shaped lid member 103 is not heat-sealed against the annular flange 124 of the container 102 and is easily peeled off in the first embodiment. In addition, a protruding portion 125a disposed at a corner portion of the heat-sealing convex portion 125 functions as a peeling start portion. A thickness of the sheet-shaped lid member 103 is preferably about 0.05 to 1.00 mm, and more preferably about 0.10 to 0.50 mm.

Incidentally, the ventilation portion is disposed on the sheet-shaped lid member 103 in the above-described first embodiment, but the invention is not limited thereto, and the ventilation portion may be disposed on the container 102.

### Industrial Applicability

The syringe assembly of the present invention is configured according to claim 1.

In this syringe assembly, the force of engagement between the cylindrical cover member and the seal cap is increased compared to before sterilization in the case of selecting the sterilization accompanied by heating such as the high-pressure steam sterilization. Thus, when the cap is removed at the time of use, only the cylindrical cover member is removed from the barrel, and it is possible to reliably remove the entire cap without the sea cap remaining on the barrel.

In addition, the above embodiment may be configured according to claims 2-11.

## Claims

1. A syringe assembly (10) comprising:
a barrel (2) that comprises a barrel body portion (21), a nozzle portion (22) disposed at a distal end of the barrel body portion (21), and a puncture needle (6) including a puncture needle tip (61) at a distal end of the puncture needle (6) and having a proximal endfixedly inserted into the nozzle portion (22); and
a cap (3) mounted to the barrel (2) and sealing the puncture needle tip (61),
wherein the cap (3) comprises:
a seal cap (7) that includes a closed distal end portion (71a), an open proximal end portion (72), a hollow portion (70) including a puncture needle storage portion storing the puncture needle (6), an insertion-allowing portion (73) into which the puncture needle tip (61) of the puncture needle (6) stored in the puncture needle storage portion is inserted, and a seal portion (75) in close contact with the nozzle portion (22) to seal the puncture needle storage portion; and
a cylindrical cover member (8) mounted at an outer side of the seal cap (7) to be engaged with the seal cap (7), and
wherein the seal cap (7) is made of an elastic material which includes water vapor permeable properties for high-pressure steam sterilization, and the cylindrical cover member (8) is made of a thermoplastic plastic material harder than the seal cap (7), and a force of engagement between the cylindrical cover member (8) and the seal cap (7) is increasable compared to before sterilization by autoclave sterilization of the syringe assembly (10), **characterized in that** the force of engagement is increased compared to before the sterilization due to deformation of the cylindrical cover member (8) caused by heat during the autoclave sterilization,
wherein the cylindrical cover member (8) includes an internal protruding portion (85) disposed on an inner surface of the cylindrical cover member (8) to be engaged with the seal cap (7), the internal protruding portion (85) being configured to be displaced inward due to heat during the autoclave sterilization such that the force of engagement is increased compared to before the sterilization.

2. The syringe assembly (10) according to claim 1, wherein the internal protruding portion (85) extends obliquely in a center axis direction and a distal end direction of the cylindrical cover member (8) and is elastically deformable.

3. The syringe assembly (10) according to any one of claims 1 to 2, wherein the cylindrical cover member (8) includes a plurality of internal protruding portions (85) disposed on an inner surface of the cylindrical cover member (8) to be engaged with the seal cap (7) and disposed at intervals about a central axis of the cylindrical cover member (8), and the cylindrical cover member (8) comprises openings extending in a distal end direction from a proximal end of each of the plurality of internal protruding portions (85).

4. The syringe assembly (10) according to any one of claims 1 to 3, wherein the cylindrical cover member (8) comprises:
a side surface covering portion (81) that covers a side surface of the seal cap (7); and
a distal end covering portion (82) that covers the closed distal end portion (71a) of the seal cap (7),
wherein the distal end covering portion (82) includes an opposing inner surface (88) that opposes a distal end surface of the closed distal end portion (71a) of the seal cap (7), and
wherein a gap (31) is formed between the opposing inner surface (88) and the closed distal end portion (71a).

5. The syringe assembly (10) according to claim 4, wherein the distal end covering portion (82) of the cylindrical cover member (8) comprises a plurality of frames (89a, 89b) connected to a distal end of the side surface covering portion (81), and
wherein a through-hole (84) is formed between the frames (89a, 89b) adjacent to each other.

6. The syringe assembly (10) according to claim 5, wherein in a side view, the cylindrical cover member (8) comprises a corner portion (82a) that is formed at a boundary between a proximal end of the frame and a distal end of the side surface covering portion (81) and that faces the distal end direction.

7. The syringe assembly (10) according to any one of claims 1 to 6, wherein the cylindrical cover member (8) includes a through-hole (84) disposed at a distal end, and
wherein the cap (3) comprises a steam guide gap (32) that is formed by an inner surface of the cylindrical cover member (8) and an outer surface of the seal cap (7), communicates with the through-hole (84), and extends to a proximal end of the puncture needle storage portion of the seal cap (7).

8. The syringe assembly (10) according to claim 7, wherein the seal portion (75) of the seal cap (7) stores the nozzle portion (22) and is in a state of being expanded outward by a distal end of the stored nozzle portion (22), and
wherein the steam guide gap (32) of the seal portion (75) has a narrower gap distance than other portions.

9. The syringe assembly (10) according to any one of claims 1 to 8, wherein the seal cap (7) is attached to the barrel (2).

10. A prefilled syringe comprising: the syringe assembly (10) according to any one of claims 1 to 9; a gasket (4) stored in the barrel (2) and slidable in the barrel in a liquid-tight manner; and a medical solution (11) filled in a space formed by the barrel (2) and the gasket (4).

11. A syringe assembly packaging (100) storing a plurality of the syringe assemblies (10) according to any one of claims 1 to 9,
the packaging (100) comprising: a container (102) having an open upper surface and having shape retainability; a barrel holding member (104) capable of holding the plurality of syringe assemblies (10); the plurality of syringe assemblies (10) held by the barrel holding member (104); a sheet-shaped lid member (103) that hermetically seals the open upper surface of the container (102) and is peelable; and a ventilation portion that is disposed on the container (102) or the lid member (103) and includes bacterial impermeable properties and sterilization gas flowability, the packaging (100) being subjected to high-pressure steam sterilization.

## Patentansprüche

1. Spritzenanordnung (10), umfassend:
einen Zylinder (2), der einen Zylinderkörperabschnitt (21), einen Düsenabschnitt (22), der an einem distalen Ende des Zylinderkörperabschnitts (21) angeordnet ist, und eine Punktionsnadel (6) umfasst, die eine Punktionsnadelspitze (61) an einem distalen Ende der Punktionsnadel (6) umfasst und die ein proximales Ende aufweist, das fest in den Düsenabschnitt (22) eingesetzt ist; und
eine Kappe (3), die am Zylinder (2) angebracht ist, und die Punktionsnadelspitze (61) abdichtet, wobei die Kappe (3) umfasst:
eine Dichtungskappe (7), die einen geschlossenen distalen Endabschnitt (71a), einen offenen proximalen Endabschnitt (72), einen hohlen Abschnitt (70), welcher einen Punktionsnadel-Aufbewahrungsabschnitt zum Aufbewahren der Punktionsnadel (6) umfasst, einen Einführungsermöglichungsabschnitt (73), in den die Punktionsnadelspitze (61) der Punktionsnadel (6), die in dem Punktionsnadel-Aufbewahrungsabschnitt aufbewahrt ist, eingeführt wird, und einem Dichtungsabschnitt (75) aufweist, der in engem Kontakt mit dem Düsenabschnitt (22) steht, um den Punktionsnadel-Aufbewahrungsabschnitt abzudichten; und
ein zylindrisches Abdeckelement (8), das an einer Außenseite der Dichtungskappe (7) angebracht ist, um mit der Dichtungskappe (7) in Eingriff zu kommen, und wobei die Dichtungskappe (7) aus einem elastischen Material hergestellt ist, das wasserdampfdurchlässige Eigenschaften für die Hochdruckdampfsterilisation aufweist, und das zylindrische Abdeckelement (8) ist aus einem thermoplastischen Kunststoffmaterial hergestellt, das härter als die Dichtungskappe (7) ist, und eine Eingriffskraft zwischen dem zylindrischen Abdeckelement (8) und der Dichtungskappe (7) kann durch eine Sterilisation der Spritzenanordnung (10) im Autoklaven im Vergleich zu vor der Sterilisation erhöht werden, **dadurch gekennzeichnet, dass** die Eingriffskraft im Vergleich zu vor der Sterilisation aufgrund einer durch Wärme während der Sterilisation im Autoklaven verursachten Verformung des zylindrischen Abdeckelements (8) erhöht wird,
wobei das zylindrische Abdeckelement (8) einen inneren Vorsprungsabschnitt (85) aufweist, der an einer Innenfläche des zylindrischen Abdeckelements (8) angeordnet ist, um mit der Dichtungskappe (7) in Eingriff zu kommen, wobei der innere Vorsprungsabschnitt (85) so ausgebildet ist, dass er sich aufgrund von Wärme während der Sterilisation im Autoklaven derart nach innen verschiebt, dass die Eingriffskraft im Vergleich zu vor der Sterilisation erhöht wird.

2. Spritzenanordnung (10) nach Anspruch 1, wobei sich der innere Vorsprungsabschnitt (85) schräg in Richtung einer Mittelachse und in Richtung des distalen Endes des zylindrischen Abdeckelements (8) erstreckt und elastisch verformbar ist.

3. Spritzenanordnung (10) nach einem der Ansprüche 1 bis 2, wobei das zylindrische Abdeckelement (8) eine Vielzahl von inneren Vorsprungsabschnitten (85) aufweist, die an einer Innenfläche des zylindrischen Abdeckelements (8) angeordnet sind, um mit der Dichtungskappe (7) in Eingriff zu kommen, und die in Abständen um eine Mittelachse des zylindrischen Abdeckelements (8) angeordnet sind, und das zylindrische Abdeckelement (8) Öffnungen aufweist, die sich von einem proximalen Ende von jedem der Vielzahl von inneren Vorsprungsabschnitten (85) in Richtung des distalen Endes erstrecken.

4. Spritzenanordnung (10) nach einem der Ansprüche 1 bis 3, wobei das zylindrische Abdeckelement (8) umfasst:
einen Seitenflächen-Abdeckabschnitt (81), der eine Seitenfläche der Dichtungskappe (7) abdeckt; und einen distalen End-Abdeckungsabschnitt (82), der den geschlossenen distalen Endabschnitt (71a) der Dichtungskappe (7) abdeckt,
wobei der distale End-Abdeckungsabschnitt (82) eine gegenüberliegende Innenfläche (88) aufweist, die einer distalen Endfläche des geschlossenen distalen Endabschnitts (71a) der Dichtungskappe (7) gegenüberliegt, und wobei zwischen der gegenüberliegenden Innenfläche (88) und dem geschlossenen distalen Endabschnitt (71a) ein Spalt (31) ausgebildet ist.

5. Spritzenanordnung (10) nach Anspruch 4, wobei der distale End-Abdeckungsabschnitt (82) des zylindrischen Abdeckelements (8) eine Vielzahl von Rahmen (89a, 89b) umfasst, die mit einem distalen Ende des die Seitenfläche abdeckenden Abschnitts (81) verbunden sind, und wobei eine Durchgangsbohrung (84) zwischen den Rahmen (89a, 89b), die benachbart zueinander sind, ausgebildet ist.

6. Spritzenanordnung (10) nach Anspruch 5, wobei in einer Seitenansicht das zylindrische Abdeckelement (8) einen Eckabschnitt (82a) aufweist, der an einer Grenze zwischen einem proximalen Ende des Rahmens und einem distalen Ende des Seitenflächen-Abdeckungsabschnitts (81) ausgebildet ist und der Richtung des distalen Endes zugewandt ist.

7. Spritzenanordnung (10) nach einem der Ansprüche 1 bis 6, wobei das zylindrische Abdeckelement (8) eine an einem distalen Ende angeordnete Durchgangsbohrung (84) aufweist und wobei die Kappe (3) einen Dampfführungsspalt (32) umfasst, der durch eine Innenfläche des zylindrischen Abdeckelements (8) und eine Außenfläche der Dichtungskappe (7) ausgebildet wird, der mit der Durchgangsbohrung (84) in Verbindung steht und sich bis zu einem proximalen Ende des Punktionsnadel-Aufbewahrungsabschnitts der Dichtungskappe (7) erstreckt.

8. Spritzenanordnung (10) nach Anspruch 7, wobei der Dichtungsabschnitt (75) der Dichtungskappe (7) den Düsenabschnitt (22) aufnimmt und sich in einem Zustand, der nach außen aufgeweitet ist, durch ein distales Ende des aufgenommenen Düsenabschnitts (22) befindet, und wobei der Dampfführungsspalt (32) des Dichtungsabschnitts (75) einen engeren Spaltabstand aufweist als andere Abschnitte.

9. Spritzenanordnung (10) nach einem der Ansprüche 1 bis 8, wobei die Dichtungskappe (7) an dem Zylinder (2) angebracht ist.

10. Fertigspritze, umfassend: die Spritzenanordnung (10) gemäß einem der Ansprüche 1 bis 9; eine Dichtung (4), die in dem Zylinder (2) aufbewahrt wird und in einer flüssigkeitsdichten Art und Weise in dem Zylinder verschiebbar ist; und eine medizinische Lösung (11), die in einen durch den Zylinder (2) und die Dichtung (4) gebildeten Raum eingefüllt ist.

11. Verpackung (100) für Spritzenanordnung, die eine Vielzahl von Spritzenanordnungen (10) nach einem der Ansprüche 1 bis 9 aufnimmt,
wobei die Verpackung (100) umfasst: einen Behälter (102), der eine offenen Oberseite aufweist und eine Formbeständigkeit aufweist; ein Zylinderhalteelement (104), das die Vielzahl der Spritzenanordnungen (10) halten kann; wobei die Vielzahl der Spritzenanordnungen (10) von dem Zylinderhalteelement (104) gehalten werden; ein blattförmiges Deckelelement (103), das die offene Oberseite des Behälters (102) hermetisch verschließt und abziehbar ist; und einen Belüftungsabschnitt, der an dem Behälter (102) oder an dem Deckelelement (103) angeordnet ist und bakterienundurchlässige Eigenschaften sowie Sterilisationsgasdurchlässigkeit aufweist, wobei die Verpackung (100) einer Hochdruckdampfsterilisation unterzogen wird.

## Revendications

1. Ensemble seringue (10) comprenant :
un cylindre (2) qui comprend une partie corps de cylindre (21), une partie embout (22) disposée à une extrémité distale de la partie corps de cylindre (21), et une aiguille pour ponction (6) comportant une pointe d'aiguille pour ponction (61) à une extrémité distale de l'aiguille pour ponction (6) et ayant une extrémité proximale insérée de manière fixe dans la partie embout (22) ; et
un capuchon (3) monté sur le cylindre (2) et assurant l'étanchéité de la pointe d'aiguille pour ponction (61),
dans lequel le capuchon (3) comprend :
un capuchon d'étanchéité (7) qui comporte une partie d'extrémité distale fermée (71a), une partie d'extrémité proximale ouverte (72), une partie creuse (70) comportant une partie de stockage d'aiguille pour ponction stockant l'aiguille pour ponction (6), une partie permettant l'insertion (73) dans laquelle la pointe d'aiguille pour ponction (61) de l'aiguille pour ponction (6) stockée dans la partie de stockage d'aiguille pour ponction est insérée, et une partie d'étanchéité (75) en contact étroit avec la partie embout (22) pour assurer l'étanchéité de la partie de stockage d'aiguille pour ponction ; et
un organe de recouvrement cylindrique (8) monté sur un côté extérieur du capuchon d'étanchéité (7) pour être mis en prise avec le capuchon d'étanchéité (7), et
dans lequel le capuchon d'étanchéité (7) est constitué d'un matériau élastique qui présente des propriétés perméables à la vapeur d'eau pour une stérilisation à la vapeur à haute pression, et l'organe de recouvrement cylindrique (8) est constitué d'un matériau plastique thermoplastique plus dur que le capuchon d'étanchéité (7), et une force de mise en prise entre l'organe de recouvrement cylindrique (8) et le capuchon d'étanchéité (7) peut augmenter par rapport à avant la stérilisation en autoclave de l'ensemble seringue (10), **caractérisé en ce que** la force de mise en prise est augmentée par rapport à avant la stérilisation en raison d'une déformation de l'organe de recouvrement cylindrique (8) provoquée par la chaleur pendant la stérilisation en autoclave,
dans lequel l'organe de recouvrement cylindrique (8) comporte une partie saillante interne (85) disposée sur une surface interne de l'organe de recouvrement cylindrique (8) pour être mise en prise avec le capuchon d'étanchéité (7), la partie saillante interne (85) étant configurée pour être déplacée vers l'intérieur sous l'effet de la chaleur pendant la stérilisation en autoclave de sorte que la force de mise en prise soit augmentée par rapport à avant la stérilisation.

2. Ensemble seringue (10) selon la revendication 1, dans lequel la partie saillante interne (85) s'étend obliquement dans une direction d'axe central et une direction d'extrémité distale de l'organe de recouvrement cylindrique (8) et est élastiquement déformable.

3. Ensemble seringue (10) selon l'une quelconque des revendications 1 à 2, dans lequel l'organe de recouvrement cylindrique (8) comporte une pluralité de parties saillantes internes (85) disposées sur une surface interne de l'organe de recouvrement cylindrique (8) pour être mises en prise avec le capuchon d'étanchéité (7) et disposées à intervalles autour d'un axe central de l'organe de recouvrement cylindrique (8), et l'organe de recouvrement cylindrique (8) comprend des ouvertures s'étendant dans une direction d'extrémité distale à partir d'une extrémité proximale de chacune de la pluralité de parties saillantes internes (85).

4. Ensemble seringue (10) selon l'une quelconque des revendications 1 à 3, dans lequel l'organe de recouvrement cylindrique (8) comprend :
une partie de recouvrement de surface latérale (81) qui recouvre une surface latérale du capuchon d'étanchéité (7) ; et
une partie de recouvrement d'extrémité distale (82) qui recouvre la partie d'extrémité distale fermée (71a) du capuchon d'étanchéité (7),
dans lequel la partie de recouvrement d'extrémité distale (82) comporte une surface interne opposée (88) qui s'oppose à une surface d'extrémité distale de la partie d'extrémité distale fermée (71a) du capuchon d'étanchéité (7), et dans lequel un espace (31) est formé entre la surface interne opposée (88) et la partie d'extrémité distale fermée (71a).

5. Ensemble seringue (10) selon la revendication 4, dans lequel la partie de recouvrement d'extrémité distale (82) de l'organe de recouvrement cylindrique (8) comprend une pluralité de cadres (89a, 89b) reliés à une extrémité distale de la partie de recouvrement de surface latérale (81), et dans lequel un trou traversant (84) est formé entre les cadres (89a, 89b) adjacents les uns aux autres.

6. Ensemble seringue (10) selon la revendication 5, dans lequel, en vue latérale, l'organe de recouvrement cylindrique (8) comprend une partie d'angle (82a) qui est formée à une limite entre une extrémité proximale du cadre et une extrémité distale de la partie de recouvrement de surface latérale (81) et qui est orientée vers la direction d'extrémité distale.

7. Ensemble seringue (10) selon l'une quelconque des revendications 1 à 6, dans lequel l'organe de recouvrement cylindrique (8) comporte un trou traversant (84) disposé à une extrémité distale, et dans lequel le capuchon (3) comprend un espace de guidage de vapeur (32) qui est formé par une surface interne de l'organe de recouvrement cylindrique (8) et une surface externe du capuchon d'étanchéité (7),
communique avec le trou traversant (84) et s'étend jusqu'à une extrémité proximale de la partie de stockage d'aiguille pour ponction du capuchon d'étanchéité (7).

8. Ensemble seringue (10) selon la revendication 7, dans lequel la partie d'étanchéité (75) du capuchon d'étanchéité (7) stocke la partie embout (22) et est dans un état d'expansion vers l'extérieur au moyen d'une extrémité distale de la partie embout (22) stockée, et
dans lequel l'espace de guidage de vapeur (32) de la partie d'étanchéité (75) présente une distance d'écartement plus étroite que d'autres parties.

9. Ensemble seringue (10) selon l'une quelconque des revendications 1 à 8, dans lequel le capuchon d'étanchéité (7) est fixé au cylindre (2).

10. Seringue préremplie comprenant : l'ensemble seringue (10) selon l'une quelconque des revendications 1 à 9 ; un joint (4) stocké dans le cylindre (2) et coulissant dans le cylindre de manière étanche aux liquides ; et une solution médicale (11) remplissant un espace formé par le cylindre (2) et le joint (4).

11. Emballage d'ensembles seringues (100) stockant une pluralité d'ensembles seringues (10) selon l'une quelconque des revendications 1 à 9,
l'emballage (100) comprenant : un récipient (102) présentant une surface supérieure ouverte et ayant une capacité de conservation de forme ; un organe de maintien de cylindre (104) apte à maintenir la pluralité d'ensembles seringues (10) ; la pluralité d'ensembles seringues (10) maintenus par l'organe de maintien de cylindre (104) ; un organe formant couvercle (103) en forme de feuille qui assure l'étanchéité hermétique de la surface supérieure ouverte du récipient (102) et est apte à être décollé ; et une partie de ventilation qui est disposée sur le récipient (102) ou l'organe formant couvercle (103) et présente des propriétés d'imperméabilité bactérienne et
une aptitude à l'écoulement de gaz de stérilisation, l'emballage (100) étant soumis à une stérilisation à la vapeur à haute pression.
